(19) Europäisches Patentamt
European Patent Office
Office européen des brevets

(11) Publication number: 0 061 774
A2

(12) EUROPEAN PATENT APPLICATION

(21) Application number: 82102682.0

(22) Date of filing: 30.03.82

(51) Int. Cl.[3]: C 07 D 213/85
C 07 D 213/73, A 61 K 31/44
//C07D213/82

(30) Priority: 30.03.81 US 248840
08.09.81 US 300294

(43) Date of publication of application:
06.10.82 Bulletin 82/40

(84) Designated Contracting States:
AT BE CH DE FR GB IT LI LU NL SE

(71) Applicant: STERLING DRUG INC.
90 Park Avenue
New York New York(US)

(72) Inventor: Lesher, George Yohe
R.D. 1 Box 268 Miller Road
East Greenbush New York(US)

(72) Inventor: Opalka, Chester Joseph, Jr.
1331 Angelo Ave. Castleton
New York(US)

(72) Inventor: Page, Donald Frederick
21 Alva Street
East Greenbush New York(US)

(74) Representative: Baillie, Iain Cameron et al,
c/o Ladas & Parry Isartorplatz 5
D-8000 München 2(DE)

# (54) 3-Amino-5-(substituted)-2 (1H) pyridinones and 3-cyano compounds useful as cardiotonics and preparation thereof.

(57) 1-$R_1$-3-amino-5-(3-R'-4-R''-phenyl)-6-R-2(1H)-pyridinones or salts thereof, which are useful as cardiotonics, where $R_1$ is hydrogen, lower-alkyl, or lower-hydroxyalkyl, R is hydrogen or lower-alkyl, and, R' and R'' are each hydrogen, hydroxy or amino at least one of R' or R'' being other than hydrogen, are prepared by reacting the corresponding 3-carbamyl compound with a reagent capable of converting carbamyl to amino or by first reacting the corresponding 3-carbamyl compound where R' and R'' are each hydrogen, nitro or lower-alkoxy with a reagent capable of converting carbamyl to amino and then reacting the resulting 3-amino compound where R' and/or R'' are/is lower-alkoxy and/or nitro with a reagent capable of converting lower-alkoxy to hydroxy and/or with a reagent capable of converting nitro to amino. Preparation of the corresponding 3-carbamyl and 3-cyano compounds are described. The invention also concerns the 3- and/or 4-methoxy intermediates and certain novel 3-cyano analogs. Cyano compounds are also useful as cardiotonics.

EP 0 061 774 A2

This invention relates to 3-amino-5-(substituted)-2(1H)-pyridinones, their preparation and use as cardiotonics.

Julia et at., Bull. soc. chim. (France), 2387-2394 (1966), show inter alia the reaction of 1-hydroxymethylene-1-phenyl-2-propanone with α-cyanoacetamide to produce 2-hydroxy-5-(unsubstituted-phenyl)-6-methylnicotinonitrile and the reaction of 3-dimethylamino-2-phenyl-2-propenal (same as β-dimethylamino-α-phenylacrolein) with α-cyanoacetamide to produce 2-hydroxy-5-(unsubstituted-phenyl)nicotinonitrile. These 2-hydroxy compounds, tautomers of the corresponding 1,2-dihydro-2-(1H)-pyridinones, were converted to their corresponding carboxylic acids and ethyl or methyl esters and also to their 2-chloro compounds and 5-(unsubstituted-phenyl)-3-piperidine-carboxamide derivatives, representative members of which were found to have pharmacological activity resembling that of lysergamide.

U.S. Patent 3,718,743 shows "5-phenyl-2-piperidinones and 5-phenyl-2-thiopiperidinones in compositions and methods for treating pain, fever and inflammation". The generic teaching of these piperidinones shows that "phenyl" can have one or two substituents at positions 2, 3, 4, 5 and/or 6, e.g., inter alia, nitro, amino, lower-alkyl, lower-alkylamino and lower-alkylmercapto. Various means of preparing the 5-phenyl-2-piperidinone final products are shown. In one procedure, a 2-chloro-5-phenylpyridine was heated with aqueous sodium hydroxide in dimethylformamide to produce the corresponding 5-phenyl-2(1H)-pyridinones which were then hydrogenated to produce the desired 5-phenyl-2-piperidinones. Among the intermediate 5-phenyl-2(1H)-

pyridinones specifically shown is 5-(4-hydroxyphenyl)-2(1H)-pyridinone and its preparation by heating the corresponding 5-(4-methoxyphenyl)-2(1H)pyridinone with pyridine hydrochloride under nitrogen.

U.S. Patents 3,655,679 and 3,703,582 show as antiinflammatory, analgesic and antipyretic agents various aryl-hydroxy-pyridinecarboxylic acids and lower-alkyl esters thereof, among which are 5-(substituted-phenyl)-2-hydroxynicotinic acids; these latter compounds were prepared by reacting a 2-(substituted-phenyl)-3-dimethylamino-2-propenal with cyanoacetamide to first produce 5-(substituted-phenyl)-2-hydroxynicotinonitrile, illustrated <u>inter alia</u> by the compounds where substituted-phenyl is 4-chlorophenyl, 3,4-dihydroxyphenyl, 4-nitrophenyl, 4-benzoylaminophenyl and 2,6-dimethoxyphenyl. Also shown is the preparation of the corresponding 2-hydroxy-6-methyl-5-(substituted-phenyl) nicotinonitrile by reacting 2-(substituted-phenyl)-acetoacetaldehyde with cyanoacetamide followed by hydrolysis of the nicotinonitrile to the corresponding nicotinic acid; illustrations of intermediate nicotinonitriles produced by this procedure include <u>inter alia</u> the compounds where substituted-phenyl is 2-hydroxyphenyl, 4-methoxyphenyl and 4-aminophenyl.

U.S. Patents 4,004,012 and 4,072,746 show as cardiotonic agents 3-amino(or cyano)-5-(pyridinyl)-2(1H)-pyridinones and as intermediates, the corresponding 3-carbamyl compounds, alternatively named 1,2-dihydro-2-oxo-5-(pyridinyl)nicotinamides, which are converted to the corresponding 3-amino compounds by reaction with a reagent capable of converting carbamyl to amino, e.g., by heating with an alkali metal hypohalite. A preferred embodiment of these compounds is 3-amino-5-(4-pyridinyl)-2(1H)-pyridinone, now generically known as amrinone and alternatively named 5-amino-[3,4'-bipyridin]-6(1H)-one. One method shown for preparing the 3-cyano-5-(pyridinyl)-2(1H)-pyridinones, alternatively named 1,2-dihydro-2-oxo-5-(pyridinyl)nicotinonitriles, is the reaction of α-(pyridinyl)-

β-(dialkylamino)acrolein with α-cyanoacetamide. U.S. Patent 4,072,746 also shows 3-Q-5-(pyridinyl)-2(1H)-pyridinones where Q is hydrogen, halo, lower-alkylamino, di-(lower-alkyl) amino and NHAc where Ac is lower-alkanoyl or lower-carbalkoxy. The disclosure of U.S. Patent 4,072,746 also is shown in U.S. Patents 4,107,315, 4,137,233, 4,199,586 and 4,225,715.

U.S. Patent 4,313,951 shows as cardiotonic agents 3-amino(or cyano or carbamyl)-6-(lower-alkyl)-5-(pyridinyl)-2(1H)-pyridinones.

Patent Cooperation Treaty Application No. 81/02575 (designating Switzerland), published September 17, 1981, and corresponding U.K. Patent Applications No. 2,070,606, published September 9, 1981, disclose, inter alia, as cardiotonic agents and claim selected 3-amino-6-$R_2$-5-aryl-2(1H)-pyridinones where $R_2$ is hydrogen or lower-alkyl and aryl is, inter alia, phenyl, 4-methoxyphenyl, 3-methoxyphenyl and 3,4-dimethoxyphenyl.

The present invention resides in a 1-$R_1$-3-amino-5-(3-R'-4-R"-phenyl)-6-R-2(1H)-pyridinone having Formula I

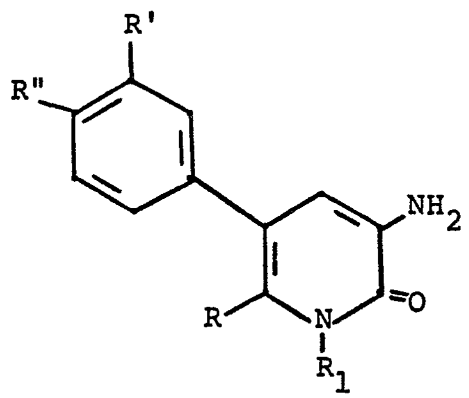

I

or an acid-addition salt thereof, where $R_1$ is hydrogen, lower-alkyl or lower-hydroxyalkyl, R is hydrogen or lower-alkyl, R' and R" are each hydrogen, amino or hydroxy at least one of R' or R" being other than hydrogen. The compounds of Formula I are useful as cardiotonics, as determined by standard pharmacological evaluation procedures. Preferred embodiments are those of Formula I where $R_1$ is hydrogen, R is methyl or ethyl, R' is hydrogen, hydroxy or amino and R" is hydroxy or hydrogen, the latter only when R' is other than hydrogen. A particularly preferred embodiment is 3-amino-5-(4-hydroxyphenyl)-6-methyl-2(1H)-pyridinone (R" is hydroxy, R' and $R_1$ are each hydrogen and R is methyl) or pharmaceutically acceptable acid-addition salt thereof.

A process for producing 1-$R_1$-3-amino-5-(3-R'-4-R"-phenyl)-6-R-2(1H)-pyridinone of Formula I comprises reacting the corresponding 1,2-dihydro-2-oxo-5-(3-R'-4-R"-phenyl)6-R-nicotinamide with a reagent capable of converting carbamyl to amino. Alternatively, the compounds of Formula I are prepared stepwise by first reacting said 1,2-dihydro-2-oxo-5-(3-R'-4-R"-phenyl)-6-R-nicotinamide where R' and/or R" instead of being hydroxy and/or amino are/is respectively lower-alkoxy and/or nitro with a reagent capable of converting carbamyl to amino and then reacting the resulting corresponding 3-amino-2(1H)-pyridinone where R' and/or R" are/is lower-alkoxy and/or nitro with a reagent capable of converting lower-alkoxy to hydroxy and/or with a reagent capable of converting nitro to amino.

The invention also resides in a 1-$R_1$-3-amino-5-(3-R'-4-R"-phenyl)-6-R-2(1H)-pyridinone having Formula I or acid-addition salt thereof, where $R_1$ is hydrogen, lower-alkyl or lower-hydroxyalkyl, R is hydrogen or lower-alkyl, R' and R" are each hydrogen or methoxy at least one of R' or R" being methoxy. These compounds are useful as intermediates for preparing the corresponding cardiotonically active compounds of Formula I where at least one of R' or R" is hydroxy. Preferred embodiments are those of Formula I where $R_1$ is hydrogen, R is methyl or ethyl, R' is hydrogen or methoxy and R" is methoxy or hydrogen, the latter only when R' is methoxy. A particularly preferred embodiment is 3-amino-5-(4-methoxyphenyl)-6-methyl-2(1H)-pyridinone or acid-addition salt thereof.

The invention also deals with a novel 1-$R_1$-1,2-dihydro-2-oxo-5-(substituted-phenyl)-6-R-nicotinonitrile of the Formula II

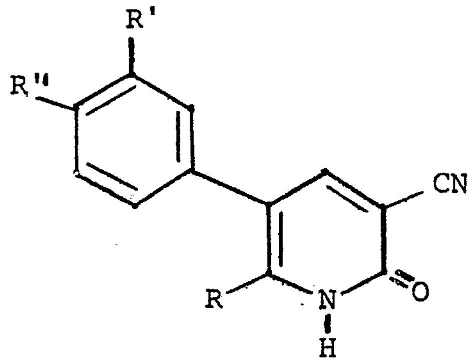

II

or an acid-addition salt thereof, wherein R" is hydroxy, R is hydrogen or lower-alkyl, and R' is hydrogen, amino, nitro or hydroxy, except that R' is not hydroxy when R is hydrogen. In addition to being useful as intermediates in preparing 1-$R_1$-1,2-dihydro-2-oxo-5-(substituted-phenyl)-6-R-nicotinonitriles used in the processes described above, these compounds have also been found to possess cardiotonic activity.

The term "lower-alkyl" as used herein, e.g., as one of the meanings of R and $R_1$, means alkyl radicals having from one to six carbon atoms which can be arranged as straight or branched chains, illustrated by methyl, ethyl, n-propyl, isopropyl, n-butyl, sec.-butyl, tert.-butyl, isobutyl, n-amyl, n-hexyl, and the like.

The term "lower-hydroxyalkyl", as used herein, e.g., as one of the meanings for $R_1$ in Formula I, means hydroxyalkyl radicals having from two to six carbon atoms and having its hydroxy group and its free valence bond (or connecting linkage) on different carbon atoms which can be arranged as straight or branched chains, illustrated by 2-hydroxyethyl, 2-hydroxypropyl, 3-hydroxypropyl, 2-hydroxy-2-methylpropyl, 2-hydroxy-1,1-dimethylethyl, 4-hydroxybutyl, 5-hydroxyamyl, 6-hydroxyhexyl, and the like.

The compounds of the invention having Formula I, and only those corresponding compounds of Formula II (both known and novel) where at least one of R" and R' is amino, in addition to being useful both in the free base form, are also useful in the form of acid-addition salts, and both forms are within the purview of the invention. The acid-addition salts are simply a more convenient form for use; and in practice, use of the salt form inherently amounts to use of the base form. The acids which can be used to prepare the acid-addition salts include

preferably those which produce, when combined with the free base, pharmaceutically acceptable salts, that is, salts whose anions are relatively innocuous to the animal organism in pharmaceutical doses of the salts, so that the beneficial cardiotonic properties inherent in the free base form of the cardiotonically active compounds of the invention are not vitiated by side effects ascribable to the anions. In practicing the invention, it is convenient to use the free base form; however, appropriate pharmaceutically acceptable salts within the scope of the invention are those derived from mineral acids such as hydrochloric acid, sulfuric acid, phosphoric acid, and sulfamic acid; and organic acids such as acetic acid, citric acid, lactic acid, tartaric acid, methanesulfonic acid, ethanesulfonic acid, benzenesulfonic acid, p-toluenesulfonic acid, cyclohexylsulfamic acid, quinic acid, and the like, giving the hydrochloride, sulfate, phosphate, sulfamate, acetate, citrate, lactate, tartrate, methanesulfonate, ethanesulfonate, benzenesulfonate, p-toluenesulfonate, cyclohexylsulfamate and quinate, respectively.

The acid-addition salts of said basic compounds are prepared either by dissolving the free base in aqueous or aqueous-alcohol solution or other suitable solvents containing the appropriate acid and isolating the salt by evaporating the solution, or by reacting the free base and acid in an organic solvent, in which case the salt separates directly or can be obtained by concentration of the solution.

Although pharmaceutically acceptable salts of said basic compounds are preferred, all acid-addition salts are within the scope of our invention. All acid-addition salts are useful as sources of the free base form even if the particular salt per se is desired only as an intermediate product as for example when the salt is formed only for purposes of purification or identification, or when it is used as an intermediate in preparing a pharmaceutically acceptable salt by ion exchange procedures.

The molecular structures of the compounds of the

invention were assigned on the basis of evidence provided by infrared, nuclear magnetic resonance and mass spectra, and by the correspondence of calculated and found values for the elemental analyses.

The manner of making and using the instant invention will now be generally described so as to enable a person skilled in the art of pharmaceutical chemistry to make and use the same, as follows.

The conversion of 1-$R_1$-1,2-dihydro-2-oxo-5-(3-R'-4-R"-phenyl)-6-R-nicotinamide to the corresponding 3-amino-1-$R_1$-5-(3-R'-4-R"-phenyl)-6-R-2(1H)-pyridinone is carried out by reacting 1-$R_1$-1,2-dihydro-2-oxo-5-(3-R'-4-R"-phenyl)-6-R-nicotinamide with a reagent capable of converting carbamyl to amino. This reaction is conveniently run by first reacting an aqueous mixture containing an alkali metal hypohalite, preferably sodium or potassium hypobromite or hypochorite, with said substituted nicotinamide at about room temperature or below, next heating the reaction mixture and then acidifying it preferably with an aqueous mineral acid, e.g., hydrochloric acid. The reaction can be carried out from about 25°C. to 100°C., preferably about 60°C. to 100°C. The compounds of Formula I also are prepared stepwise by first reacting the 1-$R_1$-1,2-dihydro-2-oxo-5-(3-R'-4-R"-phenyl)-6-R-nicotinamide where R' and/or R" instead of being hydroxy and/or amino are/is lower-alkoxy and/or nitro respectively to produce the corresponding 3-amino compound where R' and/or R" are/is lower-alkoxy and/or nitro and then converting lower-alkoxy to hydroxy and/or converting nitro to amino by generally known means as illustrated hereinbelow, e.g., Examples C-2 and C-4.

The preparation of the said intermediate 1-$R_1$-1,2-dihydro-2-oxo-5-(substituted-phenyl)-6-R-nicotinamides is carried out by generally known procedures as illustrated below in Examples B-1 through B-31, one such procedure being partial hydrolysis of the corresponding generally known 1-$R_1$-1,2-dihydro-2-oxo-5-(substituted-phenyl)-6-R-nicotinonitriles. Certain of said above 1-$R_1$-1,2-dihydro-2-oxo-5-(substituted-phenyl)-6-R-nicotinonitriles are novel and have the Formula II as defined above. The novel cardiotonically active 1,2-dihydro-2-oxo-5-(4-hydroxy-3-R'-phenyl)-6-R-2(1H)-nicotinonitriles are prepared by reacting a compound of the Formula III or IV

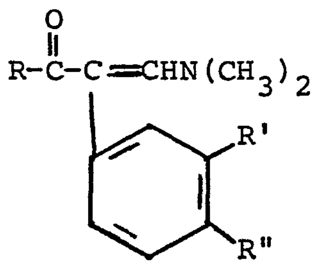

III

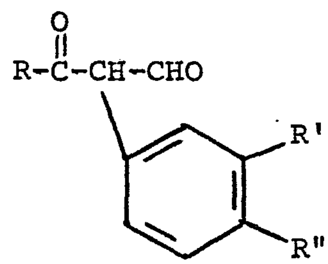

IV

where R is hydrogen or lower-alkyl, R" is hydroxy or lower-alkoxy and R' is hydrogen, amino, nitro, hydroxy or lower-alkoxy, except that R' is not hydroxy or lower-alkoxy when R is hydrogen, and, in the event of producing a compound of Formula II where R' and/or R" are/is respectively lower-alkoxy, reacting said compound obtained with a reagent capable of converting lower-alkoxy to hydroxy, and, if desired, converting a free base obtained to an acid-addition salt thereof. The reaction of a compound of Formula III with α-cyanoacetamide is generally carried out, preferably by heating at about 65 to 150°C., the reactants in a suitable solvent but in the presence of a basic condensing agent. The reaction is conveniently run using an alkali metal lower-alkoxide, preferably sodium methoxide or ethoxide, in a lower-alkanol, e.g., methanol or ethanol, or in an aprotic solvent such as dimethyl formamide. Other basic condensing agents include sodium hydride, lithium diethylamide, and the like. Other aprotic solvents include tetrahydrofuran, acetonitrile, ethyl, ether, benzene, dioxane, and the like. The reaction mixture is acidified and the product isolated. The reaction of the compound of Formula IV with α-cyanoacetamide is carried out preferably by heating at about 65 to 150°C., in the presence of a condensing agent, preferably morpholine or piperidine and/or its acetate. The reaction is conveniently carried out by refluxing a benzene solution containing the reactants in the presence

of morpholine, piperidine, morpholine acetate, piperidiene acetate or mixtures thereof, preferably with a water separator attached to the reaction vessel to collect the water produced by the reaction. The reaction also can be run using other solvents such as those shown above in the reaction of Formula III with α-cyanoacetamide. The above reactions preferably are carried out using III or IV where R" and R' are methoxy rather than hydroxy to produce the corresponding 1,2-dihydro-2-oxo-5-(4-methoxy-3-R'-phenyl or 3,4-dimethoxyphenyl)-6-R-2(1H)-nicotinonitrile and then reacting said compound with a reagent capable of converting methoxy to hydroxy, e.g., hydrogen bromide, lithium iodide and collidine, and the like.

The preparation of the 1-$R_1$-1,2-dihydro-2-oxo-5-(substituted-phenyl)-6-R-nicotinonitriles used herein is illustrated below in Examples A-1 through A-31.

The following examples will further illustrate the invention without, however, limiting it thereto.

A. 1-$R_1$-1,2-DIHYDRO-2-OXO-5-(SUBSTITUTED-PHENYL)-6-R-NICOTINONITRILES

A-1. 1,2-Dihydro-5-(4-methoxyphenyl)-2-oxo-nictotinonitrile - To a stirred mixture containing 13.5 g. of sodium methoxide in 200 ml. of methanol was added 12.61 g. of cyanoacetamide and 20.5 g. of 3-dimethylamino-2-(4-methoxyphenyl)-2-propenal and the resulting mixture was heated with stirring on a steam bath for 12 hours. The reaction mixture was concentrated *in vacuo* to yield a yellow semi-solid material. This material was taken up with water and the resulting mixture (partial dissolution) was acidified with acetic acid. The separated solid was collected, washed with water, dried *in vacuo*, recrystallized from dimethylformamide and dried *in vacuo* for 24 hours at 0.01 mm and 80°C. to produce 11.6 g. of 1,2-dihydro-5-(4-methoxyphenyl)-2-oxonicotinonitrile, m.p. 294-295°C. with decomposition.

The intermediate 3-dimethylamino-2-(4-methoxyphenyl)-2-propenal was prepared by the procedure described below in the second paragraph of Example B-5 but using α-(4-methoxyphenyl)acetic acid in place of α-(3,4-dimethoxyphenyl)acetic acid.

A-2. 1,2-Dihydro-5-(4-hydroxyphenyl)-2-oxonicotinonitrile - To a mixture containing 11.3 g. of 1,2-dihydro-5-(4-methoxyphenyl)-2-oxonicotinonitrile in 110 ml. of distilled collidine was added 36.8 g. of anhydrous lithium iodide and the resulting mixture was refluxed with stirring under nitrogen for 24 hours. The reaction mixture was cooled, treated with ice and acidified with 6N hydrochloric acid and cooled. The precipitated solid was collected and dried *in vacuo* to yield 8.5 g. of 1,2-dihydro-5-(4-hydroxyphenyl)-2-oxonicotinonitrile. The product obtained herein was combined with another sample obtained by comparable run and the combined material was recrystallized from dimethylformamide-methanol and dried at 0.01 mm. and 100°C. for 7 days to produce 13.47 g. of 1,2-dihydro-5-(4-hydroxyphenyl)-2-oxonicotinonitrile, m.p. >300°C.

A-3. 1,2-Dihydro-5-(4-nitrophenyl)-2-oxonicotinonitrile, m.p. 339-346°C. with decomposition, 87.2 g., was obtained following the procedure described in Example A-1 using 83 g. of 3-dimethylamino-2-(4-nitrophenyl)-2-propenal, 50.4 g. of cyanoacetamide, 54 g. of sodium methoxide and 1500 ml. of methanol.

A-4. 5-(4-Aminophenyl)-1,2-dihydro-2-oxonicotinonitrile - A mixture containing 14.47 g. of 1,2-dihydro-5-(4-nitrophenyl)-2-oxonicotinonitrile, 1.0 g. of 10% palladium-on-charcoal, 300 ml. of dimethylformamide and 5.76 g. (3.9 ml.) of methanesulfonic acid was treated under catalytic hydrogenation conditions until the theoretical quantity of hydrogen to reduce nitro to amino was taken up. The reaction mixture was filtered thru diatomaceous earth to remove the catalyst. The filtrate was concentrated in vacuo to a volume of about 200 ml. and to the concentrate was added slowly 3.9 ml. (5.76 g.) of methanesulfonic acid, the mixture stirred for about 10 minutes and then treated with about 500 ml. of methylene dichloride. The crystalline solid was collected, dissolved in about 900 ml. of hot methanol, the hot solution treated with decolorizing charcoal and then filtered thru a sintered glass funnel. The filtrate was cooled and the separated crystalline material was collected and recrystallized from methanol. The resulting product was dissolved in 150 ml. of warm 2N hydrochloric acid, the solution basified with aqueous sodium hydroxide solution and cooled. The resulting white solid was collected, dissolved in 400 ml. of methanol, treated with 2 ml. of methanesulfonic acid, the mixture concentrated to remove the solvent and recrystallized twice from methanol, filtering the hot yellow solution thru a sintered glass funnel and

concentrating the filtrate to a volume of about 200 ml., cooling, collecting the solid and drying it *in vacuo* at 80°C. to yield 6.1 g. of 5-(4-aminophenyl)1,2-dihydro-2-oxonicotinonitrile as its monomethanesulfonate, m.p. 252-260°C.

A-5. 1,2-Dihydro-5-(4-methoxyphenyl)-6-methyl-2-oxonicotinonitrile - A mixture containing 100 g. of 97% 3-(4-methoxyphenyl)propan-2-one, 94.2 ml. of dimethylformamide dimethyl acetal and 500 ml. of dimethylformamide was stirred at room temperature for over 17 hours and then on a steam bath for 2 hours. There was then added 23.6 ml. of dimethylformamide dimethyl acetal followed by stirring at room temperature for about 75 minutes and then on a steam bath for about 2 1/2 hours. To the partially cooled solution containing 4-dimethylamino-3-(4-methoxyphenyl)-3-buten-2-one was added 79.8 g. of sodium methoxide and 74.5 g. of cyanoacetamide and the resulting mixture was heated on a steam bath with stirring for about 12 hours. The reaction mixture was then diluted with about 1.5 liters of water and the resulting suspension was acidified with acetic acid. The separated solid was collected and recrystallized from dimethylformamide-water to yield 116.8 g. of 1,2-dihydro-5-(4-methoxyphenyl)-6-methyl-2-oxonicotinonitrile, m.p. 253-255°C. A sample of this material was further purified by recrystallizing successively from methanol and acetic acid-water and drying at 90-95°C. *in vacuo* for over 24 hours to yield said product, m.p. 258-259°C.

A-6. 1,2-Dihydro-5-(4-hydroxyphenyl)-6-methyl-2-oxonicotinonitrile, m.p. 275-276°C. with decomposition, was prepared following the procedure described in Example A-2 using 12.0 g. of 1,2-dihydro-5-(4-methoxyphenyl)-6-methyl-2-oxonicotinonitrile, 36.8 g. of lithium iodide and 120 ml. of 2,4,6-trimethylpyridine.

Following the procedure described in Example A-1 but using in place of 3-dimethylamino-2-(4-methoxyphenyl)-2-propenal a molar equivalent quantity of the appropriate 3-dimethylamino-2-(substituted-phenyl)-2-propenal, it is contemplated that the corresponding 1,2-dihydro-2-oxo-5-(substituted-phenyl)nicotinonitriles of Examples A-7 through A-11 can be obtained.

A-7. 1,2-Dihydro-5-(3-methoxyphenyl)-2-oxonicotinonitrile.

A-8. 1,2-Dihydro-5-(3,4-dimethoxyphenyl)-2-oxonicotinonitrile.

A-9. 1,2-Dihydro-5-(3-nitrophenyl)-2-oxonicotinonitrile.

A-10. 1,2-Dihydro-5-(3,4-dinitrophenyl)-2-oxonicotinonitrile.

A-11. 1,2-Dihydro-5-(4-methoxy-3-nitrophenyl)-2-oxonicotinonitrile.

The intermediate 3-dimethylamino-2-(substituted-phenyl)-2-propenals used in Examples A-7 through A-11 are either known or are readily prepared from known compounds by conventional means, e.g., by reacting an α-(substituted-phenyl)-acetic acid with the reaction product obtained by reacting dimethylformamide with a phosphorus oxyhalide, preferably the oxychloride or oxybromide, as illustrated hereinbelow in the second paragraph of Example B-5.

Following the procedure described in Example A-2

or A-4 but using in place of 1,2-dihydro-5-(4-methoxyphenyl or 4-nitrophenyl)-2-oxonicotinonitrile a molar equivalent quantity of the appropriate 1,2-dihydro-5-(substituted-phenyl)-2-oxonicotinonitrile, it is contemplated that the corresponding compounds of Examples A-12 through A-16 can be obtained.

A-12. 1,2-Dihydro-5-(3-hydroxyphenyl)-2-oxonicotinonitrile using the procedure of Example A-2.

A-13. 1,2-Dihydro-5-(3,4-dihydroxyphenyl)-2-oxonicotinonitrile using the procedure of Example A-2.

A-14. 1,2-Dihydro-5-(4-methoxy-3-aminophenyl)-2-oxonicotinonitrile using the procedure of Example A-4.

A-15. 1,2-Dihydro-5-(3-aminophenyl)-2-oxonicotinonitrile using the procedure of Example A-4.

A-16. 1,2-Dihydro-5-(3,4-diaminophenyl)-2-oxonicotinonitrile using the procedure of Example A-4.

Following the procedure described in Example A-5 but using in place of 3-(4-methoxyphenyl)propan-2-one a molar equivalent quantity of the appropriate 3-(substituted-phenyl)propan-2-one or substituted-benzyl lower-alkyl ketone, it is contemplated that the corresponding 1,2-dihydro-2-oxo-5-(substituted-phenyl)-6-methyl(or lower-alkyl)-nicotinonitriles of Examples A-17 to A-21 can be obtained.

A-17. 1,2-Dihydro-5-(3-methoxyphenyl)-6-methyl-2-oxonicotinonitrile, m.p. 247-250°C., starting with 3-(3-methoxyphenyl)propan-2-one.

A-18. 1,2-Dihydro-5-(3,4-dimethoxyphenyl)-6-methyl-2-oxonicotinonitrile, m.p. 262-263°C., starting with 3-(3,4-dimethoxyphenyl)propan-2-one via 4-dimethylamino-3-(3,4-dimethoxyphenyl)-3-buten-2-one, m.p. 94-95.5°C. (prepared as in Example A-5).

A-19. 1,2-Dihydro-5-(4-nitrophenyl)-6-methyl-2-oxonicotinonitrile, starting with 3-(4-nitrophenyl) propan-2-one.

A-20. 1,2-Dihydro-5-(4-methoxyphenyl)-6-ethyl-2-oxonicotinonitrile, starting with 1-(4-methoxyphenyl) butan-2-one.

A-21. 1,2-Dihydro-5-(4-methoxy-3-nitrophenyl)-6-methyl-2-oxonicotinonitrile, m.p. >300°C., starting with 3-(4-methoxy-3-nitrophenyl)propan-2-one.

Following the procedure described in Example A-2 or A-4 but using in place of 1,2-dihydro-5-(4-methoxyphenyl or 4-nitrophenyl)-2-oxonicotinonitrile a molar equivalent quantity of the appropriate 1,2-dihydro-5-(substituted-phenyl)-6-(lower-alkyl)-2-oxonicotinonitrile, it is contemplated that the corresponding compounds of Examples A-22 through A-27 can be obtained.

A-22. 1,2-Dihydro-5-(3-hydroxyphenyl)-6-methyl-2-oxonicotinonitrile, using 1,2-dihydro-5-(3-methoxyphenyl)-6-methyl-2-oxonicotinonitrile and the procedure of Example A-2.

A-23. 1,2-Dihydro-5-(3,4-dihydroxyphenyl)-6-methyl-2-oxonicotinonitrile, m.p. 267°C. (chars), using 1,2-dihydro-5-(3,4-dimethoxyphenyl)-6-methyl-2-oxonicotinonitrile and the procedure of Example A-2.

A-24. 1,2-Dihydro-5-(4-aminophenyl)-6-methyl-2-oxonicotinonitrile, using 1,2-dihydro-5-(4-nitrophenyl)-6-methyl-2-oxonicotinonitrile and the procedure of Example A-4.

A-25. 1,2-Dihydro-5-(4-hydroxyphenyl)-6-ethyl-2-oxonicotinonitrile, using 1,2-dihydro-5-(4-methoxyphenyl)-6-ethyl-2-oxonicotinonitrile and the procedure of Example A-2.

A-26. 1,2-Dihydro-5-(4-hydroxy-3-nitrophenyl)-6-methyl-2-oxonicotinonitrile, m.p. 290-293°C., using 1,2-dihydro-5-(4-methoxy-3-nitrophenyl)-6-methyl-2-oxonicotinonitrile and the procedure of Example A-2.

A-27. 1,2-Dihydro-5-(3-amino-4-hydroxyphenyl)-6-methyl-2-oxonicotinonitrile, using 1,2-dihydro-5-(4-hydroxy-3-nitrophenyl)-6-methyl-2-oxonicotinonitrile and the procedure of Example A-4.

Following the procedure described in Example A-5 but using in place of cyanoacetamide a molar equivalent quantity of the appropriate N-$R_1$-cyanoacetamide, it is

contemplated that the corresponding 1-$R_1$-1,2-dihydro-5-(4-methoxyphenyl)-6-R-2-oxonicotinonitrile of Examples A-28 and A-29 can be obtained.

A-28. 1,2-Dihydro-5-(4-methoxyphenyl)-1,6-dimethyl-2-oxonicotinonitrile, using N-methylcyanoacetamide.

A-29. 1,2-Dihydro-1-(2-hydroxyethyl)-5-(4-methoxy-phenyl)-6-methyl-2-oxonicotinonitrile, using N-(2-hydroxyethyl)cyanoacetamide.

Following the procedure described in Example A-2 but using in place of 1,2-dihydro-5-(4-methoxyphenyl)-2-oxonicotinonitrile a molar equivalent quantity of the appropriate 1-$R_1$-1,2-dihydro-5-(4-methoxyphenyl)-6-(lower-alkyl)-2-oxonicotinonitrile, it is contemplated that the corresponding compounds of Examples A-30 and A-31 can be obtained.

A-30. 1,2-Dihydro-5-(4-hydroxyphenyl)-1,6-dimethyl-2-oxonicotinonitrile.

A-31. 1,2-Dihydro-1-(2-hydroxyethyl)-5-(4-hydroxyphenyl) -6-methyl-2-oxonicotinonitrile.

B. <u>1-$R_1$-1,2-DIHYDRO-2-OXO-5-(SUBSTITUTED-PHENYL)-6-R-NICOTINAMIDES</u>

B-1. <u>1,2-Dihydro-5-(4-methoxyphenyl)-2-oxonicotinamide</u> - A mixture containing 82.1 g. of 3-dimethylamino-2-(4-methoxyphenyl)-2-propenal, 63.15 g. of malonamide (97%), 54.0 g. of sodium methoxide and 800 ml. of methanol was refluxed with stirring for about 17 hours and chilled. The reaction mixture was filtered and the filtrate concentrated *in vacuo* to a volume of about 500 ml., acidified with acetic acid and chilled. The resulting solid was collected, dried at 90°C. in a vacuum oven to yield 25.25 g. of 1,2-dihydro-5-(4-methoxyphenyl)-2-oxonicotinamide, m.p. 283-286°C.

B-2. <u>1,2-Dihydro-5-(4-nitrophenyl)-2-oxonicotinamide</u> - A mixture containing 1,2-dihydro-5-(4-nitrophenyl)-2-oxonicotinonitrile and 1 liter of 90% sulfuric acid was stirred at room temperature for about 3 hours, allowed to stand at room temperature overnight (about 15 hours) and

then stirred on a steam bath for about 2 hours. The reaction mixture was partially cooled and then quenched in a mixture of 2.5 liters of ice-water. The solid that separated was collected, washed with water, suspended in 5% aqueous potassium carbonate solution sufficient to impart weak alkalinity to the suspension. The suspension was stirred and the solid was collected, washed with water and dried at 90°C. in a vacuum oven to yield 103.7 g. of 1,2-dihydro-5-(4-nitrophenyl)-2-oxonicotinamide, m.p. >340°C.

B-3. 5-(4-Aminophenyl)-1,2-dihydro-2-oxonicotinamide - A mixture containing 1,2-dihydro-5-(4-nitrophenyl)-2-oxonicotinamide, 200 ml. of acetic acid and 0.20 g. of platinum dioxide was catalytically hydrogenated at room temperature for a period of 20 hours. The reaction mixture was filtered and the filter cake washed well with warm dimethylformamide; the filter cake was saved for further treatment described below. The combined acetic acid filtrate and dimethylformamide washings were concentrated *in vacuo*. The residue was taken up in isopropyl alcohol, the solution was acidified with hydrogen chloride in ethanol and then was diluted with ether and cooled. The separated product was collected and dried as below to yield 3.44 g. of 5-(4-aminophenyl)-1,2-dihydro-2-oxonicotinamide as its hydrochloride, m.p. >315°C. More product was obtained from the original filter cake (*supra*) by successively extracting it with hydrogen chloride in warm ethanol and then hot dimethylformamide. To the combined extracts was added excess ethanolic-hydrogen chloride, the solution was concentrated to a volume of less than 150 ml. and diluted with ether to precipitate the product; this was collected and dried in a vacuum oven at 90°C. to produce another 3.72 g. of 5-(4-aminophenyl)-1,2-dihydro-2-oxonicotinamide, m.p. >315°C. The combined samples of product, (7.16 g.), were dissolved in dilute hydrochloric acid and the solution treated with 10% potassium bicarbonate solution. The separated product was collected and dried at 90°C. in a vacuum oven to produce 1.07 g. of 5-(4-aminophenyl)-1,2-dihydro-2-oxonicotinamide containing 1/4 mole of water per mole of product, m.p. >315°C.

B-4. 1,2-Dihydro-5-(4-methoxyphenyl)-6-methyl-2-oxonicotinamide - A 20.0 g. portion of 1,2-dihydro-5-(4-methoxyphenyl)-6-methyl-2-oxonicotinonitrile was added to 200 g. of polyphosphoric acid and the resulting mixture was heated with stirring on a steam bath overnight. The reaction mixture was added with stirring to about 200 ml. of ice-water mixture. The resulting mixture was made alkaline with ammonium hydroxide. The resulting solid was collected, air-dried, recrystallized from dimethylformamide and dried *in vacuo* at 100°C. and 0.01 mm. to yield 9.0 g. of 1,2-dihydro-5-(4-methoxyphenyl)-6-methyl-2-oxonicotinamide, m.p. >300°C.

B-5. 1,2-Dihydro-5-(3,4-dimethoxyphenyl)-2-oxonicotinamide - A mixture containing 235 g. of 2-(3,4-dimethoxyphenyl)-3-dimethylamino-2-propenal, 2 liters of methanol, 108 g. of sodium methoxide and 150 g. of malonamide was refluxed with stirring for about 30 minutes and the reaction mixture heated *in vacuo* to remove the solvent. The residue was dissolved in water and neutralized with acetic acid. The separated solid was collected, washed with water, dried, recrystallized from dimethylformamide and dried at 70°C. to yield 81 g. of 1,2-dihydro-5-(3,4-dimethoxyphenyl)-2-oxonicotinamide. A 20 g. sample of the material was recrystallized a second time from dimethylformamide to yield 16 g. of the product, m.p. 266-268°C.

The intermediate 2-(3,4-dimethoxyphenyl)-3-dimethylamino-2-propenal was prepared as follows: To a chilled 1260 ml. portion of dimethylformamide was added dropwise 230 ml. of phosphorus oxychloride followed by addition of 196 g. of α-(3,4-dimethoxyphenyl)acetic acid. The reaction mixture was heated at about 70°C. on a steam bath for 3 hours, allowed to cool and then concentrated *in vacuo* to remove the solvent and excess volatile reactants. The resulting product was used directly in the above procedure.

Following the procedure described in Example B-1 but using in place of 3-dimethylamino-2-(4-methoxyphenyl)-2-propenal a molar equivalent quantity of the appropriate 3-dimethylamino-2-(substituted-phenyl)-2-propenal, it is contemplated that the corresponding 1,2-dihydro-2-oxo-5-(substituted-phenyl)nicotinamides of Examples B-6 through B-10 can be obtained.

B-6. 1,2-Dihydro-5-(3-methoxyphenyl)-2-oxonicotinamide.

B-7. 1,2-Dihydro-5-(3,4-diethoxyphenyl)-2-oxonicotinamide.

B-8. 1,2-Dihydro-5-(3-nitrophenyl)-2-oxonicotinamide.

B-9. 1,2-Dihydro-5-(3,4-dinitrophenyl)-2-oxonicotinamide.

B-10. 1,2-Dihydro-5-(4-methoxy-3-nitrophenyl)-2-oxonicotinamide.

Following the procedure described in Example A-2 or B-3 but using in place of 1,2-dihydro-5-(4-methoxyphenyl)-2-oxonicotinonitrile or 1,2-dihydro-5-(4-nitrophenyl)-2-oxonicotinamide respectively a molar equivalent quantity of the appropriate 1,2-dihydro-5-(substituted-phenyl)-2-oxonicotinamide, it is contemplated that the corresponding compounds of Examples B-11 through B-15 can be obtained.

B-11. 1,2-Dihydro-5-(3-hydroxyphenyl)-2-oxonicotinamide using the procedure of Example A-2.

B-12. 1,2-Dihydro-5-(3,4-dihydroxyphenyl)-2-oxonicotinamide using the procedure of Example A-2.

B-13. 1,2-Dihydro-5-(4-methoxy-3-aminophenyl)-2-oxonicotinamide using the procedure of Example B-3.

B-14. 1,2-Dihydro-5-(3-aminophenyl)-2-oxonicotinamide using the procedure of Example B-3.

B-15. 1,2-Dihydro-5-(3,4-diaminophenyl)-2-oxonicotinamide using the procedure of Example B-3.

Following the procedure described in Example B-4 but using in place of 1,2-dihydro-5-(4-methoxyphenyl)-6-methyl-2-oxonicotinonitrile a molar equivalent quantity of the appropriate 1,2-dihydro-5-(substituted-phenyl)-6-(lower-alkyl)nicotinonitrile, it is contemplated that the

corresponding 1,2-dihydro-2-oxo-5-(substituted-phenyl)-6-(lower-alkyl)nicotinamides of Examples B-16 through B-22 can be obtained.

B-16. 1,2-Dihydro-5-(3-methoxyphenyl)-6-methyl-2-oxonicotinamide, m.p. 298-300°C.

B-17. 1,2-Dihydro-5-(3,4-dimethoxyphenyl)-6-methyl-2-oxonicotinamide, m.p. >300°C.

B-18. 1,2-Dihydro-5-(4-nitrophenyl)-6-methyl-2-oxonicotinamide.

B-19. 1,2-Dihydro-5-(4-methoxyphenyl)-6-ethyl-2-oxonicotinamide.

B-20. 1,2-Dihydro-5-(4-methoxy-3-nitrophenyl)-6-methyl-2-oxonicotinamide.

B-21. 1,2-Dihydro-5-(4-hydroxy-3-nitrophenyl)-6-methyl-2-oxonicotinamide.

B-22. 1,2-Dihydro-5-(3-amino-4-hydroxyphenyl)-6-methyl-2-oxonicotinamide.

Following the procedure described in Example A-2 or B-3 but using in place of 1,2-dihydro-5-(4-methoxyphenyl)-2-oxonicotinonitrile or 1,2-dihydro-5-(4-nitrophenyl)-2-oxonicotinamide respectively a molar equivalent quantity of the appropriate 1,2-dihydro-5-(substituted-phenyl)-6-(lower-alkyl)-2-oxonicotinamide, it is contemplated that the corresponding compounds of Examples B-23 through B-27 can be obtained.

B-23. 1,2-Dihydro-5-(3-hydroxyphenyl)-6-methyl-2-oxonicotinamide.

B-24. 1,2-Dihydro-5-(3,4-dihydroxyphenyl)-6-methyl-2-oxonicotinamide.

B-25. 1,2-Dihydro-5-(4-aminophenyl)-6-methyl-2-oxonicotinamide.

B-26. 1,2-Dihydro-5-(4-hydroxyphenyl)-6-ethyl-2-oxonicotinamide.

B-27. 1,2-Dihydro-5-(3-amino-4-hydroxyphenyl)-6-methyl-2-oxonicotinamide.

Following the procedure described in Example B-2 but using in place of 1,2-dihydro-5-(4-nitrophenyl)-2-oxonicotinonitrile a molar equivalent quantity of the appropriate 1-$R_1$-1,2-dihydro-5-(substituted-phenyl)-6-R-2-oxonicotinonitrile, it is contemplated that the corresponding 1-$R_1$-1,2-dihydro-5-(substituted-phenyl)-6-R-2-oxonicotinamides of Examples B-28 through B-31 can be obtained.

B-28. 1,2-Dihydro-5-(4-methoxyphenyl)-1,6-dimethyl-2-oxonicotinamide.

B-29. 1,2-Dihydro-1-(2-hydroxyethyl)-5-(4-methoxyphenyl)—6-methyl-2-oxonicotinamide.

B-30. 1,2-Dihydro-5-(4-hydroxyphenyl)-1,6-dimethyl-2-oxonicotinamide.

B-31. 1,2-Dihydro-1-(2-hydroxyethyl)-5-(4-hydroxyphenyl)—6-methyl-2-oxonicotinamide.

C. 1-$R_1$-3-AMINO-5-(SUBSTITUTED-PHENYL)6-R-2(1H)-PYRIDINONES

C-1. 3-Amino-5-(4-methoxyphenyl)-2(1H)-pyridinone - To a cold solution containing 19.6 g. of sodium hydroxide in 450 ml. of water was added over a 20 minute period 4.92 ml. of bromine and the resulting mixture was stirred for about 15 additional minutes. To this solution containing sodium hypobromite was added 19.54 g. of 1,2-dihydro-5-(4-methoxyphenyl)-2-oxonicotinamide. The resulting reaction was stirred at room temperature for about 2 hours and then on a steam bath for an additional hour. The partially cooled suspension was acidified carefully with 6N hydrochloric acid and then neutralized with 2N potassium hydroxide solution. The resulting suspension was chilled and the solid product was collected by filtration. The solid was boiled with 600 ml. of isopropyl alcohol and the resulting solution diluted with 600 ml. of dimethylformamide. The resulting hot solution was concentrated to a volume 500 ml. and the resulting suspension was acidified with hydrogen chloride in ethanol. The resulting mixture was stirred, diluted with ether and the solid was collected by filtration and dried in a vacuum oven at 90°C. to yield 14.5 g. of product, m.p. 242-246°C. A 5.64 g. portion of the product was dissolved in dimethylformamide and the solution treated with hydrogen chloride in ethanol to acidity. The separated product was collected and dried *in vacuo* in a vacuum oven at 90°C. to yield 3.92 g. of 3-amino-5-(4-methoxyphenyl)-2(1H)-pyridinone as its hydrochloride, m.p. 238-242°C. with decomposition.

C-2. 3-Amino-5-(4-hydroxyphenyl)-2(1H)-pyridinone - A mixture containing 7.58 g. of 3-amino-5-(4-methoxyphenyl)-2(1H)-pyridinone, 90 ml. of 48% hydrogen bromide and 180 ml. of acetic acid was refluxed with stirring for about 8 hours. To the reaction mixture was added 108.9 g. of sodium acetate trihydrate in 300 ml. of water. The resulting suspension was stirred and then concentrated *in vacuo* to remove volatile materials. The remaining residue was mixed well with water; the solid material was collected

by filtration and washed with water. The solid was taken up in about 300 ml. of water and the aqueous mixture was treated with an excess of 2N potassium hydroxide solution and the resulting mixture filtered through diatomaceous earth. The filtrate was acidified with acetic acid, the separated solid was collected and dried at 100°C. in a vacuum oven to produce 6 g. of 3-amino-5-(4-hydroxyphenyl)-2(1H)-pyridinone, m.p. 322-326°C. with decomposition.

Acid-addition salts of 3-amino-5-(4-hydroxyphenyl)-2(1H)-pyridinone are conveniently prepared by adding to a mixture of 1 g. of 3-amino-5-(4-hydroxyphenyl)-2(1H)-pyridinone in about 20 ml. of aqueous methanol the appropriate acid, e.g., hydrochloric acid, methanesulfonic acid, sulfuric acid, to a pH of about 2-3, chilling the mixture after partial evaporation and collecting the precipitated salt, e.g., hydrochloride, methanesulfonate, sulfate, respectively. Also, the lactate or hydrochloride acid-addition salt of 3-amino-5-(4-hydroxyphenyl)-2(1H)-pyridinone is conveniently prepared in aqueous solution by adding to water with stirring molar equivalent quantities each of 3-amino-5-(4-hydroxyphenyl)-2(1H)-pyridinone and lactic acid or hydrochloric acid, respectively.

C-3. <u>3-Amino-5-(4-nitrophenyl)-2(1H)-pyridinone</u>, m.p. 260-267°C. as its monohydrochloride 1/4 hydrate, 14.77 g., was prepared following the procedure described in Example C-1 using 25.92 g. of 1,2-dihydro-5-(4-nitrophenyl)-2-oxonicotinamide, 24.0 g. of sodium hydroxide, 6.15 ml. of bromine and 600 ml. of water.

C-4. <u>3-Amino-5-(4-aminophenyl)-2(1H)-pyridinone</u> - A mixture containing 10.7 g. of 3-amino-5-(4-nitrophenyl)-2(1H)-pyridinone as its monohydrochloride, 1 g. of 10% palladium-on-charcoal and 300 ml. of dimethylformamide was treated under catalytic hydrogenation conditions, the reaction mixture was filtered to remove the catalyst and the filtrate was acidified with 100 ml. of 1.4M ethanolic hydrogen chloride. The solvent was distilled off *in vacuo* and the residue was treated with hot isopropyl alcohol. The

insoluble material was collected, dissolved in about 300 ml. of methanol, treated with decolorizing charcoal, filtered and the filtrate concentrated to a volume of about 150 ml. The concentrated solution was cooled in ice and the separated product was collected and dried to yield 5.70 g. of 3-amino-5-(4-aminophenyl)-2(1H)pyridinone as its dihydrochloride dihydrate, m.p. 280-287°C. with decomposition.

C-5. 3-Amino-5-(4-methoxyphenyl)-6-methyl-2(1H)-pyridinone, m.p. 241-243°C. with decomposition as its 1/4 hydrate, was prepared following the procedure described above in Example C-1 using 22.9 g. of 1,2-dihydro-5-(4-methoxyphenyl)-6-methyl-2-oxonicotinamide, 23.9 g. sodium hydroxide dissolved in 450 ml. of water and 5.6 ml. of bromine and purification via its N-acetyl derivative described as follows. A total of 26.7 g. of 3-amino-5-(4-methoxyphenyl)-6-methyl-2(1H)-pyridinone prepared in several runs (and containing a small amount of unreacted 3-carbamyl starting material as determined by nmr) was refluxed with stirring with a mixture of 270 ml. of pyridine and 13.6 ml. of acetic anhydride. The reaction mixture was cooled, diluted with about 500 ml. of ether and the solid that separated was collected by filtration, washed with ether and dried *in vacuo* to yield 20.13 g. of 3-acetamido-5-(4-methoxyphenyl)-6-methyl-2(1H)-pyridinone. A 19.75 g. portion of said 3-acetamido compound in 200 ml. of 6N hydrochloric acid was refluxed with stirring for 32 hours (some ether cleavage here; see below). The reaction mixture was filtered and the filtrate made alkaline with ammonium hydroxide solution while cooling. The separated solid was collected by filtration, washed with water and dried. The solid was then taken up in about 100 ml. of 10% aqueous sodium hydroxide solution and the mixture stirred at room temperature for about 1 hour. The solid was collected, washed with water and dried *in vacuo*. The basic filtrate thus obtained was saved and used hereinbelow in Example C-6. The solid was again stirred with 100 ml. of 10% sodium hydroxide solution at room temperature for about 3 hours and again collected,

washed with water and dried *in vacuo* to produce 5.31 g. of 3-amino-5-(4-methoxyphenyl)-6-methyl-2(1H)-pyridinone as its 1/4 hydrate, m.p. 241-243°C. with decomposition.

C-6. 3-Amino-5-(4-hydroxyphenyl)-6-methyl-2(1H)-pyridinone - The basic filtrate obtained from the previous example, C-5, was acidified with glacial acetic acid and cooled. The solid was collected by filtration, washed with water, dried *in vacuo* and then again taken up in about 100 ml. of 10% aqueous sodium hydroxide solution. The mixture was filtered to remove a small amount of insoluble material and the filtrate was acidified with glacial acetic acid. The solid that separated was collected by filtration, washed with water and dried *in vacuo* to yield 2.56 g. of 3-amino-5-(4-hydroxyphenyl)-6-methyl-2(1H)-pyridinone, m.p. 256-257°C. with decomposition.

Acid-addition salts of 3-amino-5-(4-hydroxyphenyl)-6-methyl-2(1H)-pyridinone are conveniently prepared by adding to a mixture of 1 g. of 3-amino-5-(4-hydroxyphenyl)-6-methyl-2(1H)-pyridinone and about 20 ml. of aqueous methanol the appropriate acid, e.g., hydrochloric acid, methanesulfonic acid, sulfuric acid, to a pH of about 2-3, chilling the mixture after partial evaporation and collecting the precipitated salt, e.g., hydrochloride, methanesulfonate, sulfate, respectively. Also, the lactate or hydrochloride acid-addition salt of 3-amino-5-(4-hydroxyphenyl)-6-methyl-2(1H)-pyridinone is conveniently prepared in aqueous solution by adding to water with stirring molar equivalent quantities each of 3-amino-5-(4-hydroxyphenyl)-6-methyl-2(1H)-pyridinone and lactic acid or hydrochloric acid, respectively.

C-7. 3-Amino-5-(3,4-dimethoxyphenyl)-2(1H)-pyridinone, m.p. 208-215°C., 5 g., was prepared following the procedure described in Example C-1 using 14 g. of 1,2-dihydro-5-(3,4-dimethoxyphenyl)-2-oxonicotinamide, 270 ml. of water, 12 g. of sodium hydroxide and 3.1 ml. of bromine.

C-8. 3-Amino-5-(3,4-dihydroxyphenyl)-2(1H)pyridinone, m.p. 271-274°C. as its hemihydrate, 11 g., was prepared following the procedure described in Example C-2

using 36 g. of 3-amino-5-(3,4-dimethoxyphenyl)-2(1H)-pyridinone, 180 ml. of 48% of hydrogen bromide, 400 ml. of acetic acid and recrystallization from dimethylformamide.

Following the procedure described in Example C-1 but using in place of 1,2-dihydro-5-(4-methoxyphenyl)-2-oxonicotinamide a molar equivalent quantity of the appropriate 1,2-dihydro-5-(substituted-phenyl)-2-oxonicotinamide, it is contemplated that the corresponding 3-amino-5-(substituted-phenyl)-2(1H)-pyridinones of Examples C-9 through C-13 can be obtained.

C-9. 3-Amino-5-(3-methoxyphenyl)-2(1H)-pyridinone.

C-10. 3-Amino-5-(3,4-diethoxyphenyl)-2(1H)-pyridinone.

C-11. 3-Amino-5-(3-nitrophenyl)-2(1H)-pyridinone.

C-12. 3-Amino-5-(3,4-dinitrophenyl)-2(1H)-pyridinone.

C-13. 3-Amino-5-(4-methoxy-3-nitrophenyl)-2(1H)-pyridinone.

Following the procedure described in Example C-2 or C-4 but using in place of 3-amino-5-(4-methoxyphenyl)-2(1H)-pyridinone or 3-amino-5-(4-nitrophenyl)-2(1H)-pyridinone a molar equivalent quantity of the appropriate 3-amino-5-(methoxy- or nitrophenyl)-2(1H)-pyridinone, it is contemplated that the corresponding 3-amino-5-(hydroxy- or aminophenyl)—2(1H)-pyridinones of Examples C-14 through C-19 can be obtained.

C-14. 3-Amino-5-(3-hydroxyphenyl)-2(1H)-pyridinone, using the procedure of Example C-2.

C-15. 3-Amino-5-(3,4-dihydroxyphenyl)-2(1H)-pyridinone, using the procedure of Example C-2.

C-16. 3-Amino-5-(4-methoxy-3-aminophenyl)-2-oxonicotinonitrile using the procedure of Example C-4.

C-17. 3-Amino-5-(3-aminophenyl)-2(1H)-pyridinone, using the procedure of Example C-4.

C-18. 3-Amino-5-(3,4-diaminophenyl)-2(1H)-pyridinone, using the procedure of Example C-4.

C-19. 3-Amino-5-(4-hydroxy-3-aminophenyl)-2(1H)-pyridinone, using the procedure of Example C-2.

Following the procedure described in Example C-1 but using in place of 1,2-dihydro-5-(4-methoxyphenyl)-2-oxonicotinamide a molar equivalent quantity of the appropriate 1,2-dihydro-5-(substituted-phenyl)-6-(lower-alkyl)-2-oxonicotinamide, it is contemplated that the corresponding 3-amino-5-(substituted-phenyl)-6-(lower-alkyl)-2(1H)-pyridinones of Examples C-20 to C-23 can be obtained.

C-20. 3-Amino-5-(3-methoxyphenyl)-6-methyl-2(1H)-pyridinone, m.p. 243-245°C.

C-21. 3-Amino-5-(3,4-dimethoxyphenyl)-6-methyl-2(1H)-pyridinone, m.p. 222-224°C.

C-22. 3-Amino-5-(4-nitrophenyl)-6-methyl-2(1H)-pyridinone.

C-23. 3-Amino-5-(4-methoxyphenyl)-6-ethyl-2(1H)-pyridinone.

Following the procedure described in Example C-2 or C-4 but using in place of 3-amino-5-(4-methoxy- or 4-nitrophenyl)-2(1H)-pyridinone a molar equivalent quantity of the appropriate 3-amino-5-(methoxy- or nitrophenyl)-2(1H)-pyridinone, it is contemplated that the corresponding compounds of Examples C-24 through C-27 can be obtained.

C-24. 3-Amino-5-(3-hydroxyphenyl)-6-methyl-2(1H)-pyridinone, m.p. 251-253°C.

C-25. 3-Amino-5-(3,4-dihydroxyphenyl)-6-methyl-2(1H)-pyridinone, m.p. 260°C. (chars).

C-26. 3-Amino-5-(4-aminophenyl)-6-methyl-2(1H)-pyridinone.

C-27. 3-Amino-5-(4-hydroxyphenyl)-6-ethyl-2(1H)-pyridinone.

Following the procedure described in Example C-1 but using in place of 1,2-dihydro-5-(4-methoxyphenyl)-2-oxonicotinamide a molar equivalent quantity of the appropriate 1-$R_1$-1,2-dihydro-5-(substituted-phenyl)-6-R-2-oxonicotinamide, it is contemplated that the corresponding 3-amino-1-$R_1$-5-(substituted-phenyl)-6-R-2(1H)-pyridinones of Examples

C-28 through C-31 can be obtained.

C-28. 3-Amino-5-(4-methoxyphenyl)-1,6-dimethyl-2(1H)-pyridinone.

C-29. 3-Amino-1-(2-hydroxyethyl)-5-(4-methoxyphenyl)-6-methyl-2(1H)-pyridinone.

C-30. 3-Amino-5-(4-hydroxyphenyl)-1,6-dimethyl-2(1H)-pyridinone.

C-31. 3-Amino-1-(2-hydroxyethyl)-5-(4-hydroxyphenyl)-6-methyl-2(1H)-pyridinone.

The usefulness of the compounds of Formulas I and II or salts thereof as cardiotonic agents is demonstrated by their effectiveness in standard pharmacological test procedures, for example, in causing a significant increase in contractile force of the isolated cat or guinea pig atria and papillary muscle and/or in causing a significant increase in the cardiac contractile force in the anesthetized dog with low or minimal changes in heart rate and blood pressure. Detailed descriptions of these test procedures appear in U.S. Patent 4,072,746.

When tested by said isolated cat or guinea pig atria and papillary muscle procedure, the compounds of Formula I or pharmaceutically acceptable acid-addition salts thereof at doses of 10, 30, and/or 100 µg/ml., were found to cause significant increases, that is, greater than 25% (cat) or 30% (g. pig) in papillary muscle force and significant increases, that is, greater than 25% (cat) or 30% (g. pig) in right atrial force, while causing a lower percentage increase (about one-half or less than the precentage increase in right atrial force or papillary muscle force) in right atrial rate. Because of the lower control active tensions of guinea pig tissues the percent change from control values of both rate and force responses is elevated slightly, i.e., 5%. Thus, whereas cardiotonic activity is ascertained with a papillary muscle force or right atrial force increase of 26% and greater in the cat test, corresponding activity in the guinea pig test is designated with a papillary muscle force or right atrial force increase of 31% or greater. The

compounds of the invention (Formula I) have varying degrees of cardiotonic activity. For example, the particularly preferred embodiment of Example C-6 was found to cause guinea pig papillary muscle force and right atrial force increases of 110% and 109% at a dose level as low as 10 µg/ml. Other embodiments, e.g., Examples C-2, C-4 and C-8 had significant activity at 100 µg/ml in the cat atria and papillary muscle test procedure but not at said lower doses, i.e., at 10 and 30 µg/ml.

Illustrative cat or guinea pig papillary muscle force and right atrial force increases, respectively, for 3-cyano compounds when tested at 10 µg/ml are given as follows: Example A-2 (cat), 67% and 25%; Example A-6 (g. pig), 73% and 82%; Example A-23 (g. pig), 133% and 71%; and, Example A-26 (g. pig), 121% and 72%.

When tested by said anesthetized dog procedure, the compounds of Formula I or pharmaceutically acceptable acid-addition salts thereof at doses of 1.0, 3.0 and/or 10 mg/kg. administered intravenously were found to cause significant increases, that is, 25% or greater, in cardiac contractile force or cardiac contractility with lower changes in heart rate and blood pressure. For example, when tested at one or more of said dose levels by this procedure, the compound of Example C-2 was found to cause increases of 34 to 156% in contractile force and lower changes in heart rate and blood pressure.

A cardiotonic composition for increasing cardiac contractility comprises a pharmaceutically acceptable carrier and, as the active component thereof, a cardiotonically effective amount of the compound of Formula I or II or pharmaceutically acceptable acid-addition salt thereof. One can increase cardiac contractility in a patient requiring such treatment by administering to such patient said cardiotonic composition providing a cardiotonically effective amount of said compound of Formula I or II or pharmaceutically acceptable acid-addition salt thereof. In clinical practice said compound or salt thereof will normally be administered

orally or parenterally in a wide variety of dosage forms.

Solid compositions for oral administration include compressed tablets, pills, powders and granules. In such solid compositions, at least one of the active compounds is admixed with at least one inert diluent such as starch, calcium carbonate, sucrose or lactose. These compositions can also contain additional substances other than inert diluents, e.g., lubricating agents, such as magnesium stearate, talc and the like.

Liquid compositions for oral administration include pharmaceutically acceptable emulsions, solutions, suspensions, syrups and elixirs containing inert diluents commonly used in the art, such as water and liquid paraffin. Besides inert diluents such compositions can also contain adjuvants, such as wetting and suspending agents, and sweetening, flavoring, perfuming and preserving agents. According to the invention, the compounds for oral administration also include capsules of absorbable material, such as gelatin, containing said active component with or without the addition of diluents or excipients.

Preparations according to the invention for parenteral administration include sterile aqueous, aqueous-organic, and organic solutions, suspensions and emulsions. Examples of organic solvents or suspending media are propylene glycol, polyethylene glycol, vegetable oils such as olive oil and injectable organic esters such as ethyl oleate. These compositions can also contain adjuvants such as stabilizing, preserving, wetting, emulsifying and dispersing agents.

They can be sterilized, for example, by filtration through a bacteria-retaining filter, by incorporation of sterilizing agents in the compositions, by irradiation or by heating. They can also be manufactured in the form of sterile solid compositions which can be dissolved in sterile water or some other sterile injectable medium immediately before use.

The percentages of active component in the said composition and method for increasing cardiac contractility

can be varied so that a suitable dosage is obtained. The dosage administered to a particular patient is variable, depending upon the clinician's judgement using as the criteria: the route of administration, the duration of treatment, the size and condition of the patient, the potency of the active component and the patient's response thereto. An effective dosage amount of active component can thus only be determined by the clinician considering all criteria and utilizing the best judgement on the patient's behalf.

Another aspect of the invention resides in a cardiotonic composition for increasing cardiac contractility, said composition comprising a pharmaceutically acceptable carrier and, as the active component thereof, a cardiotonically effective amount of 1-$R_1$-3-Q-5-(3-R'-4-R"-phenyl)-6-R-2(1H)-pyridinone having the Formula IIA

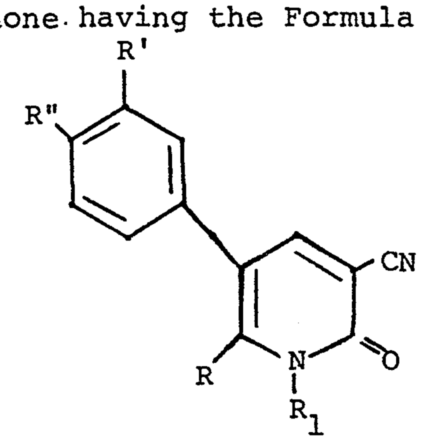

IIA

or pharmaceutically acceptable acid-addition salt thereof (when at least one of R" and R' is amino), where R, $R_1$, R' and R" have the definitions given for the novel compounds of Formula I. The compounds of Formula IIA include both the novel compounds of Formula II, as defined above, and known compounds, not previously disclosed as having cardiotonic activity. Preferred embodiments of the above cardiotonic composition of the invention are those where the active component is the compound of Formula II where $R_1$ is hydrogen, R is hydrogen, methyl or ethyl, R' is hydrogen, hydroxy or amino, and R" is hydroxy or hydrogen, the latter only when R' is other than hydrogen. Particularly preferred embodiments are those where the active component is the compound of Formula IIA where R is hydrogen or methyl, $R_1$ and Q are hydrogen and one of or both R" and R' represent

hydroxy.

The above-defined compounds of Formula IIA can be used in the method for increasing cardiac contractility in a patient requiring such treatment which comprises administering orally or parenterally in a solid or liquid dosage form to such patient a composition comprising a pharmaceutically acceptable carrier and, as active component thereof, a cardiotonically-effective amount of said 1-$R_1$-3-Q-5-(3-R'-4-R"-phenyl)-6-R-2(1H)-pyridinone of Formula IIA.

1. A compound having the Formula I (herein) or an acid-addition salt thereof, where $R_1$ is hydrogen, lower-alkyl or lower-hydroxyalkyl, R is hydrogen or lower-alkyl, and, R' and R" are each hydrogen, amino or hydroxy at least one of R' or R" being other than hydrogen.

2. A compound according to claim 1, where $R_1$ is hydrogen, R is methyl or ethyl, R' is hydrogen, hydroxy or amino and R" is hydroxy or hydrogen, the latter only when R' is other than hydrogen.

3. 3-Amino-5-(4-hydroxyphenyl)-6-methyl-2(1H)-pyridinone or a pharmaceutically acceptable acid-addition salt thereof.

4. A process for producing a compound according to claim 1 or 2, which comprises reacting a corresponding 1,2-dihydro-2-oxo-5-(3-R'-4-R"-phenyl)-6-R-nicotinamide, or such a compound where R' and/or R" instead of being hydrogen and/or amino are/is respectively lower-alkoxy and/or nitro with a reagent capable of converting carbamyl to amino, and, in the event of producing a compound of Formula I where R' and/or R" are/is lower-alkoxy and/or nitro from said latter compound, reacting said compound obtained with a reagent capable of converting lower-alkoxy to hydroxy and/or with a reagent capable of converting nitro to amino, and, if desired, converting a free base obtained into an acid-addition salt thereof.

5. A compound of the Formula II (herein) where R is hydrogen or lower-alkyl R" is hydroxy, and R' is hydrogen, amino, nitro or hydroxy, except that R' is not hydroxy when R is hydrogen, or an acid-addition salt thereof when R' is amino.

6. 1,2-Dihydro-5-(4-hydroxyphenyl)-6-methyl-2-oxonicotinonitrile.

7. A process for preparing a compound according to claim 5, which comprises reacting a compound of the Formula III or IV (herein) with α-cyanoacetamide, where R is hydrogen or lower-alkyl, R" is hydroxy or lower-alkoxy and R' is hydrogen, amino, nitro, hydroxy or lower-alkoxy, except that

R' is not hydroxy or lower-alkoxy when R is hydrogen, and, in the event of producing a compound of Formula II where R' and/or R" are/is respectively lower-alkoxy, reacting said compound obtained with a reagent capable of converting lower-alkoxy to hydroxy, and, if desired, converting a free base obtained when R' is amino to an acid-addition salt thereof.

8. A cardiotonic composition for increasing cardiac contractility, said composition comprising a pharmaceutically acceptable carried and, as the active component thereof, a cardiotonically effective amount of 1-$R_1$-3-Q-5-(3-R'-4-R"-phenyl)-6-R-2(1H)-pyridinone of the Formula IIA (herein) or pharmaceutically acceptable acid-addition salt thereof (when at least one of R" and R' is amino), where $R_1$ is hydrogen, lower-alkyl or lower-hydroxyalkyl, R is hydrogen or lower-alkyl, and, R' and R" are each hydrogen, amino or hydroxy, at least one of R' and R" being other than hydrogen, or a compound according to any one of claims 1-3, 5 and 6, or prepared according to claim 4 or 7.

9. A compound having the Formula I (herein) or an acid-addition salt thereof, where $R_1$ is hydrogen, lower-alkyl or lower-hydroxyalkyl, R is hydrogen or lower-alkyl, and, R' and R" are each hydrogen or methoxy at least one of R' or R" being methoxy.

10. A compound according to claim 9, where $R_1$ is hydrogen, R is methyl or ethyl, R' is hydrogen or methoxy and R" is methoxy or hydrogen, the latter only when R' is methoxy, e.g., 3-amino-5-(4-methoxyphenyl)-6-methyl-2(1H)-pyridinone or acid-addition salt thereof.

CLAIMS

1. A process for producing a compound having the Formula I (herein), or an acid-addition salt thereof, where $R_1$ is hydrogen, lower-alkyl or lower-hydroxyalkyl, R is hydrogen or lower-alkyl, and, R' and R" are each hydrogen, amino or hydroxy at least one of R' or R" being other than hydrogen, characterized by reacting a corresponding 1,2-dihydro-2-oxo-5-(3-R'-4-R"-phenyl)-6-R-nicotinamide, or such a compound where R' and/or R" instead of being hydrogen and/or amino are/is respectively lower-alkoxy and/or nitro with a reagent capable of converting carbamyl to amino, and, in the event of producing a compound of Formula I where R' and/or R" are/is lower-alkoxy and/or nitro from said latter compound, reacting said compound obtained with a reagent capable of converting lower-alkoxy to hydroxy and/or with a reagent capable of converting nitro to amino, and, if desired, converting a free base obtained into an acid-addition salt thereof.

2. A process according to claim 1, characterized by the fact that $R_1$ is hydrogen, R is methyl or ethyl, R' is hydrogen, hydroxy or amino and R" is hydroxy or hydrogen, the latter only when R' is other than hydrogen.

3. A process according to claim 1, for preparing 3-amino-5-(4-hydroxyphenyl)-6-methyl-2(1H)-pyridinone or a pharmaceutically acceptable acid-addition salt thereof.

4. A process for preparing a compound of the Formula II (herein) where R is hydrogen or lower-alkyl R" is hydroxy, and R' is hydrogen, amino, nitro or hydroxy, except that R' is not hydroxy when R is hydrogen, or an acid-addition salt thereof when $R^1$ is amino, characterized by reacting a compound of the Formula III or IV (herein) with α-cyanoacetamide, where R is hydrogen or lower-alkyl, R" is hydroxy or lower-alkoxy and R' is hydrogen, amino, nitro, hydroxy or lower-alkoxy, except that R' is not hydroxy or lower-alkoxy when R is hydrogen, and, in the event of producing a compound of Formula II where R' and/or R" are/is respectively lower-alkoxy, reacting said compound obtained

with a reagent capable of converting lower-alkoxy to hydroxy, and, if desired, converting a free base obtained where R' is amino to an acid-addition salt thereof.

5. A process according to claim 4, for preparing 1,2-dihydro-5-(4-hydroxyphenyl)-6-methyl-2-oxonicotinonitrile.

6. A process for preparing a compound having the Formula I (herein), or an acid-addition salt thereof, where $R_1$ is hydrogen, lower-alkyl or lower-hydroxyalkyl, R is hydrogen or lower-alkyl, and, R' and R" are each hydrogen or methoxy at least one of R' or R" being methoxy, characterized by reacting a corresponding 1-$R_1$-1,2-dihydro-2-oxo-5-(3-R'-4-R"-phenyl)-6-R-nicotinamide with a reagent capable of converting carbamyl to amino, and, if desired, converting a free base obtained into an acid-addition salt thereof.

7. A process according to claim 6, characterized by the fact that $R_1$ is hydrogen, R is methyl or ethyl, R' is hydrogen or methoxy and R" is methoxy or hydrogen, the latter only when R' is methoxy.

8. A process according to claim 6, for preparing 3-amino-5-(4-methoxyphenyl)-6-methyl-2(1H)-pyridinone or acid-addition salt thereof.